# EUROPEAN PATENT APPLICATION

(11) **EP 2 345 655 A1**
(43) Date of publication of application: **20.07.2011**
(21) Application number: 10150097.3
(22) Date of filing: 05.01.2010
(51) Int. Cl.: C07D 487/04

(54) **A process for racemisation of 6-(5-chloropyridin-2-yl)-7-(4-methyl-1-piperazinyl)carbonyloxy-7-oxo-6,7-dihydro-5H-pyrrolo[3,4-b]pyrazine**

(71) Applicant: LEK Pharmaceuticals d.d., 1526 Ljubljana (SI)
(72) Inventor: The designation of the inventor has not yet been filed

(57) **Abstract**

The present invention provides a new process of recovering zopiclone from unwanted enantiomer by racemisation using aliphatic amines in amide solvents. The process of the present invention can give product in high yield with low amount of impurities and can be carried out on an industrial scale. The present invention provides also a method for resolving the enantiomers of zopiclone by means of chiral chromatography using stationary phase which comprises amylose tris (3,5-dimethylphenylcarbamate) immobilised on silica gel.

## Description

### A process for racemisation of 6-(5-chloropyridin-2-yl)-7-(4-methyl-1-piperazinyl)carbonyloxy-7-oxo-6,7-dihydro-5H-pyrrolo[3,4-b]pyrazine

### Field of the invention

The present invention belongs to the field of organic chemistry and relates to a process for the preparation of (S)-zopiclone including racemisation of unwanted enantiomer of zopiclone into racemic zopiclone.

### Description of the background art

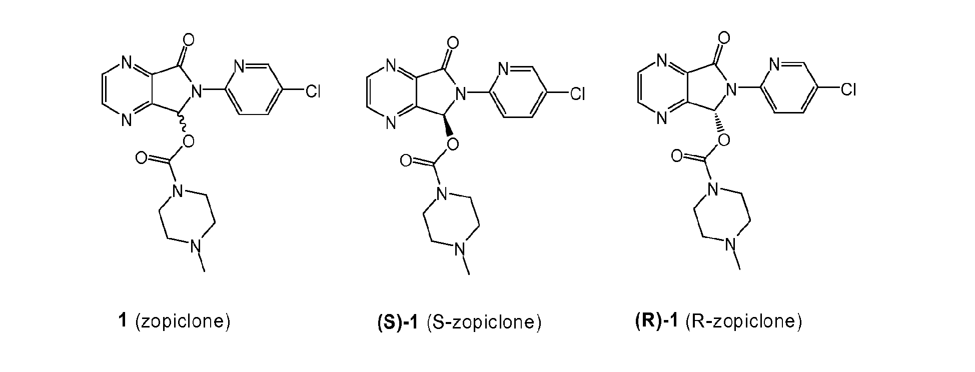

6-(5-Chloropyridin-2-yl)-7-(4-methyl-1-piperazinyl)carbonyloxy-7-oxo-6,7-dihydro-5H-pyrrolo[3,4-b]pyrazine (**1**, zopiclone) is a cyclopyrrolone with hypnotic activity. Racemic form **1** ((±)-zopiclone) has been commercialised for a long time, but later it was found that its S-enantiomer was twice as active as the racemate whereas the R-enantiomer is almost inactive but responsible from the majority of the adverse effects. The (+)-enantiomer (S)-zopiclone (generic name eszopiclone) was commercialised recently.

The enantiomers of zopiclone can be separated by crystallisation of the diastereoisomeric salts obtained with optically active acids. For example, D-malic acid (Chirality, 1993, 5, 419) or (+)-O,O'-dibenzoyltartaric acid (US Pat. 6,319,926) can be used as chiral acids for the separation of (S)-zopiclone. The theoretical yield of such separation is 50 %, but due to losses in crystallisation the practical yield of (S)-zopiclone is much lower and falls bellow 35 % of total zopiclone.

Another method of chiral separation, which is found in the literature, applied a chiral chromatography method called "simulating moving bed" using stationary phases comprising silica gel coated with a functionalized polysaccaride (WO 2006/136866), but again half of zopiclone was lost in the form of (R)-zopiclone.

Yet another improvement was applied using enzymatic resolution of carbonate intermediates of zopiclone (Tetrahedron Asymmetry 1997, 8, 995 and 2002, 13, 2577). Again half of the intermediate was returned a step aback while the wished enantiomer was transformed to zopiclone in a step in which again some additional material was lost.

Patent application WO 2000/69442 mentioned a possibility of racemisation of single enantiomer using DBU (1,8-diazabicyclo[5.4.0]undec-7-ene) or tertiary amines but it was discovered that yields of recycling of unwished enantiomers were low.

An improved racemisation of (R)-zopiclone using DBU is described in the patent application WO 2009/002552.

According to the processes disclosed in patent applications WO 2008/126105 and WO 2009/063486 (R)-zopiclone is converted to the intermediate compound 6-(5-chloropyrid-2-yl)-5-hydroxy-7-oxo-5,6-dihydropyrrolo [3,4-b]pyrazine, which is reused in the synthesis of racemic zopiclone.

Therefore there is still a need for an improved process for an isolation of eszopiclone from racemic zopiclone and an improved process for racemisation of an enantiomer of zopiclone or enantiomerically enriched mixture of (R)- and (S)- enantiomers of zopiclone.

### Summary of the invention

The present invention provides the following aspects, subject matters and preferred embodiments, which respectively taken alone or in combination, contribute to solving the object of the present invention:
(1) A process for racemisation of an enantiomer of zopiclone or enantiomerically enriched mixture of (R)- and (S)- enantiomers of zopiclone by treating with an aliphatic amine in an amide solvent or in a mixture of amide solvent and a second solvent.
(2) A process according to the item (1), wherein the enantiomer of zopiclone is (R)-zopiclone.
(3) A process according to the item (1), wherein the enantiomerically enriched mixture of both enantiomers of zopiclone comprises an excess of (R)-zopiclone.
(4) A process according to any of the preceding items, wherein the aliphatic amine is selected from the group consisting of mono-, di- or tri-C₁-C₁₂ alkyl substituted amines in which substituents are the same or different, chained or branched, separated or connected to a cycle as they are but not limited in pyrrolidines, piperidines, piperazines, morpholines, unsubstituted or substituted with aryl, or O, N or S containing groups or more cycles, preferably the aliphatic amine is mono- or di-C₁-C₁₂ alkyl substituted amine.
(5) A process according to any of the preceding items, wherein the aliphatic amine is selected from *tert*-butylamine, isobutylamine, piperazine, piperidine, sec-butylamine, dipropylamine, diisopropylamine, isopropylamine, preferably the aliphatic amine is isopropylamine.
(6) A process according to any of the preceding items, wherein the molar ratio of the aliphatic amine and zopiclone enantiomer(s) is 20:0.2, preferably 10:0.5 mol, more preferably 2:0.8, most preferably 1:1.
(7) A process according to any of the preceding items, wherein the amide solvent is selected from N,N-dimethylformamide, N, N-dimethylacetamide, 1,3-dimethyl-3,4,5,6-tetrahydro-2(1 H)-pyrimidinone, 1,3-dimethyl-2-imidazolidinone, 1-methyl-2-pyrrolidone, preferably the amide solvent is N,N-dimethylformamide.
(8) A process according to any of the preceding items, wherein the second solvent is an aprotic solvent.
(9) A process according to any of the items (1) to (8), wherein the second solvent is selected from ethanol, isopropanol, ethyl acetate, dichloromethane, diethyl ether, diisopropyl ether, methyl *tert*-butyl ether, water.
(10) A process according to any of the preceding items, wherein the process is carried out at temperature from 40 °C to 140 °C, preferably from 70 °C to 120 °C, more preferably from 85 °C to 95 °C.
(11) A process for preparing (S)-zopiclone including the process for racemisation of an enantiomer of zopiclone or enantiomerically enriched mixture of (R)- and (S)- enantiomers of zopiclone according to any of the preceding items.
(12) A process for racemisation of (R)-zopiclone comprising the following steps:
   a) providing a material comprising (R)-zopiclone,
   b) treating the material with an aliphatic amine in an amide solvent or in a mixture of amide solvent and a second solvent,
   c) isolating a racemic zopiclone.
(13) A process according to the item (12), wherein the material comprising (R)-zopiclone used in step a) is recovered from (R)-zopiclone fractions obtained after chromatographic chiral separation of racemic zopiclone.
(14) A process according to the item (13), wherein the chromatographic chiral separation of racemic zopiclone is carried out on a chiral stationary phase with functionalized polysaccharide, preferably amylose tris (3,5-dimethylphenylcarbamate).
(15) A process according to the item (14), wherein the chiral stationary phase is silica gel immobilised with functionalized polysaccharide, preferably amylose tris (3,5-dimethylphenylcarbamate).
(16) A process according to any of the items (14) to (15), wherein the mobile phase used in the chromatographic chiral separation comprises a C₁-C₆ alcohol, tetrahydrofuran, methyl *tert*-butyl ether, dichloromethane or a mixture thereof or a mixture of a C₁-C₆ alcohol with water. Preferably, C₁-C₆ alcohol is selected from methanol, ethanol, 2-propanol. Preferably, the mobile phase used in the chromatographic chiral separation is 96% ethanol.
(17) A process according to the item (12), wherein the material comprising (R)-zopiclone used in step a) is recovered from a mother liquor obtained after separation of (S)-zopiclone by crystallisation of its diasteroisomeric salt.
(18) A process according to the item (17), wherein the mother liquor is enriched in (R)-zopiclone diasteroisomeric salt.
(19) A process according to the item (17) or the item (18), wherein (R)-zopiclone diasteroisomeric salt in mother liquor is converted to (R)-zopiclone free base before carried out step b).
(20) A process according to any of the items (12) to (19), wherein the aliphatic amine is selected from the group consisting of tri-, di- or mono-C₁-C₁₂ alkyl substituted amines in which substituents are the same or different, chained or branched, separated or connected to a cycle as they are but not limited in pyrrolidines, piperidines, piperazines, morpholines, unsubstituted or substituted with aryl, or O, N or S containing groups or more cycles, preferably the aliphatic amine is di- or mono-C₁-C₁₂ alkyl substituted amine.
(21) A process according to any of the items (12) to (20), wherein the aliphatic amine is selected from *tert*-butylamine, isobutylamine, piperazine, piperidine, *sec*-butylamine, dipropylamine, diisopropylamine, isopropylamine, preferably the aliphatic amine is isopropylamine.
(22) A process according to any of the items (12) to (21), wherein the molar ratio of the aliphatic amine and zopiclone enantiomer(s) is 20:0.2, preferably 10:0.5 mol, more preferably 2:0.8, most preferably 1:1.
(23) A process according to any of the of the items (12) to (22), wherein the amide solvent is selected from N,N-dimethylformamide, N, N-dimethylacetamide, 1,3-dimethyl-3,4,5,6-tetrahydro-2(1H)-pyrimidinone, 1,3-dimethyl-2-imidazolidinone, 1-methyl-2-pyrrolidone, preferably the amide solvent is is N,N-dimethylformamide.
(24) A process according to any of the items (12) to (23), wherein the second solvent is an aprotic solvent.
(25) A process according to any of the items (12) to (23), wherein the second solvent is selected from ethanol, isopropanol, ethyl acetate, dichloromethane, diethyl ether, diisopropyl ether, methyl *tert*-butyl ether, water.
(26) A process according to any of the items (12) to (25), wherein the step b) is carried out at temperature from 40 °C to 140 °C, preferably from 70 °C to 120 °C, more preferably from 85 °C to 95 °C.
(27) A process according to any of the items (12) to (26), wherein in the step c) the racemic zopiclone is isolated by the cooling of the reaction mixture obtained in step b) and the evaporation of the solvent.
(28) A process according to any of the items (12) to (26), wherein the step c) comprises the following sub-steps:
   c1) optionally, concentrating the reaction mixture obtained in step b) by the evaporation of the solvent,
   c2) adding the reaction mixture obtained in step b) or concentrated reaction mixture obtained in step b) to water or C₁-C₆ alkyl acetates, preferably ethyl acetate; or adding water or C₁-C₆ alkyl acetates, preferably ethyl acetate, to the reaction mixture obtained in step b) or concentrated reaction mixture obtained in step b) c3) isolating the racemic zopiclone, preferably by filtration.
(29) A process according to any of the items (12) to (26), wherein the reaction mixture in step b comprises water and step c) comprises the following sub-steps:
   c1) extraction with water immiscible solvent, preferably selected from C₁-C₆ alkyl acetates, preferably ethyl acetate, and chlorinated C₁-C₆ alkanes, preferably dichloromethane,
   c2) isolating the racemic zopiclone, preferably by removing the said water immiscible solvent.
(30) A process according to any of the items (12) to (29), wherein isolated racemic zopiclone is further purified by crystallisation from C₁-C₆ alcohol, preferably methanol.
(31) A process for preparing (S)-zopiclone comprising a process for racemisation of (R)-zopiclone or a mixture of (R)- and (S)- enantiomers of zopiclone enriched in (R)-zopiclone according to any of the items (1) to (30).
(32) A continuous process for preparing (S)-zopiclone comprising the following steps:
   a) separation of racemic zopiclone,
   b) isolation of (S)-zopiclone
   c) collecting the remaining zopiclone from step a) and b) in a form of (R)-zopiclone or a mixture of (R)- and (S)- enantiomers of zopiclone enriched in (R)-zopiclone
   d) racemisation of the collected (R)-zopiclone or mixture of (R)- and (S)- enantiomers of zopiclone enriched in (R)-zopiclone by treating with an aliphatic amine in an amide solvent or in a mixture of amide solvent and a second solvent according to any of the items (1) to (30),
   e) optionally repeating step d),
   f) add recovered racemic zopiclone obtained after step d) or step e) to the fresh racemic zopiclone in step a).
(33) A process according to the item (32), wherein the separation of racemic zopiclone is carried out by chromatographic chiral separation.
(34) A process according to the item (33), wherein the chromatographic chiral separation of racemic zopiclone is carried out on a chiral stationary phase with functionalized polysaccharide, preferably amylose tris (3,5-dimethylphenylcarbamate).
(35) A process according to the item (34), wherein the chiral stationary phase is silica gel immobilised with functionalized polysaccharide, preferably amylose tris (3,5-dimethylphenylcarbamate).
(36) A process according to any of the items (34) to (35), wherein the mobile phase used in the chromatographic chiral separation comprises a C₁-C₆ alcohol, tetrahydrofuran, methyl *tert*-butyl ether, dichloromethane or a mixture thereof or a mixture of a C₁-C₆ alcohol with water. Preferably, C₁-C₆ alcohol is selected from methanol, ethanol, 2-propanol. Preferably, the mobile phase used in the chromatographic chiral separation is 96% ethanol.
(37) A process according to any of the items (32) to (36), wherein (S)-zopiclone is isolated by evaporation of mobile phase solvent from (S)-zopiclone fractions, preferably under reduced pressure at room temperature.
(38) A process according to the item (32), wherein the step a) comprises the following sub-steps:
   a1) reacting racemic zopiclone with a chiral acid, preferably selected from D-malic acid, O,O-substituted tartaric acid, N-substituted amino acids, more preferably selected from D-malic acid, O,O-dibenzoyltartaric acid, Z-phenylalanine, most preferably D-malic acid,
   a2) isolation of (S)-zopiclone diasteroisomeric salt obtained in sub-step a1) by crystallisation,
   a3) conversion of (S)-zopiclone diasteroisomeric salt to (S)-zopiclone free base.
(39) A process according to any of the items (32) to (38), wherein in step f) preferably between 5 and 45 %, more preferably between 20 and 30 % of recovered racemic zopiclone is used in a total batch of racemic zopiclone.
(40) A process for the separation of racemic zopiclone by means of chromatographic chiral separation, wherein a chiral stationary phase is silica gel immobilised with functionalized polysaccharide, preferably amylose tris (3,5-dimethylphenylcarbamate).
(41) A process according to the item (40), wherein the mobile phase used in the chromatographic chiral separation comprises a C₁-C₆ alcohol, tetrahydrofuran, methyl tert-butyl ether, dichloromethane or a mixture thereof or a mixture of a C₁-C₆ alcohol with water. Preferably, C₁-C₆ alcohol is selected from methanol, ethanol, 2-propanol. Preferably, the mobile phase used in the chromatographic chiral separation is 96% ethanol.
(42) A process according to the item (40) or the item (41), wherein (S)-zopiclone is isolated by evaporation of mobile phase solvent from (S)-zopiclone fractions, preferably under reduced pressure at room temperature.

### Detailed description of the invention

The present invention in one aspect represents a process of treating a compound with formula 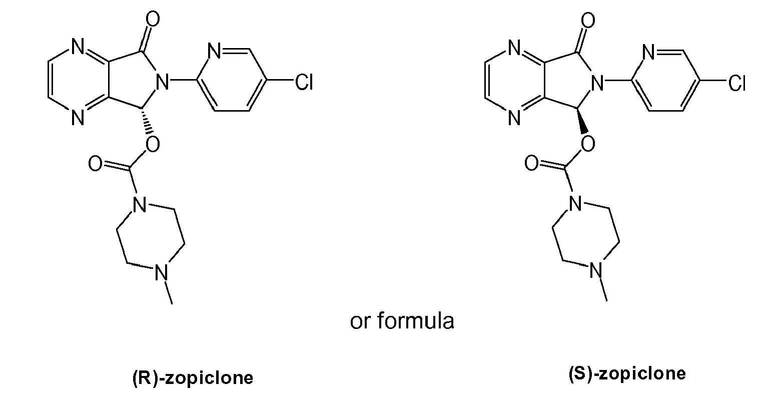 with an organic base in amide solvents at elevated temperature in order to convert the said enantiomer into the opposite one to diminish enantiomeric access or to achieve racemic mixture. Preferably, the convertible compound is (R)-isomer which is transformed by the process of our invention into the pharmaceutically applicable (S)-isomer called (S)-zopiclone or eszopiclone. (R)-zopiclone is the unwanted component of zopiclone if zopiclone is used for preparation of (S)-zopiclone. In order to reduce costs it is highly desirable to convert unusable enantiomer into the active pharmaceutical ingredient.

It is known for a skilled person that chiral compounds can racemise in basic conditions if chiral center includes a proton with sufficient acidity but it is also known that carbamates are easily hydrolysed in alkaline conditions. Hydroxides and water basic solutions easily cleave zopiclone into 6-(5-chloropyrid-2-yl)-5-hydroxy-7-oxo-5,6-dihydropyrrolo[3,4-b]pyrazine (**2**), so they are not suitable for racemisation.

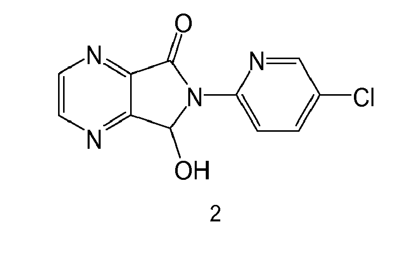

Organic bases are another possibility for racemisation, but its use is limited because the chiral conversion is slow and many impurities are formed. Surprisingly, we found out that primary and secondary amines are superior over tertiary amines or stronger organic bases like DBU despite weaker basicity and stronger nucleophylicity which could potentially cleave carbamate if the conversion is carried out in amide solvents.

Thus an enantiomer of zopiclone or enantiomerically enriched mixture of both enantiomers is dissolved or diluted in an amide solvent or in a mixture of amide solvent and second solvent, preferably another aprotic solvent, treated with an amine and the conversion is carried out at elevated temperature until the ratio of enantiomer is approximately equal or the conversion stops. The reaction mixture is then cooled and the solvent is evaporated. Optionally the reaction mixture itself or the mixture after partial concentration is poured into the water or diethyl ether and the product is isolated by filtration. Another alternative to precipitation is extraction of the product with water immiscible solvent with final isolation by removing the said solvent. The isolated product is a racemate or a mixture of enantiomers with slight excess of the starting isomer. This product which is optionally purified in order to remove impurities can be used again in the separation of enantiomers, either alone or as a mixture with originally synthesized zopiclone.

One aspect of the present invention is a process for racemisation of an enantiomer of zopiclone or enantiomerically enriched mixture of (R)- and (S)- enantiomers of zopiclone by treating with an aliphatic amine in an amide solvent or in a mixture of amide solvent and a second solvent.

The preferred enantiomer which is subjected to racemisation is (R)-zopiclone.

In a preferred embodiment the present invention relates to a process for racemisation of (R)-zopiclone or a mixture of (R)- and (S)- enantiomers of zopiclone enriched in (R)-zopiclone by treating with an aliphatic amine in an amide solvent or in a mixture of amide solvent and a second solvent.

Another aspect of the present invention is a process for racemisation of (R)-zopiclone comprising the following steps:
a) providing a material comprising (R)-zopiclone,
b) treating the material with an aliphatic amine in an amide solvent or in a mixture of amide solvent and a second solvent,
c) isolating a racemic zopiclone.
   Another aspect of the present invention is a continuous process for preparing (S)-zopiclone comprising the following steps:
   a) separation of racemic zopiclone,
   b) isolation of (S)-zopiclone,
   c) collecting the remaining zopiclone from step a) and b) in a form of (R)-zopiclone or a mixture of (R)- and (S)- enantiomers of zopiclone enriched in (R)-zopiclone
   d) racemisation of the collected (R)-zopiclone or mixture of (R)- and (S)- enantiomers of zopiclone enriched in (R)-zopiclone by treating with an aliphatic amine in an amide solvent or in a mixture of amide solvent and a second solvent,
   e) optionally repeating step d),
   f) add recovered racemic zopiclone obtained after step d) or step e) to the fresh racemic zopiclone in step a).

Another aspect of the present invention is a continuous process for preparing (S)-zopiclone comprising the following steps:
a) separation of racemic zopiclone,
b) isolation of (S)-zopiclone,
c) collecting the remaining zopiclone from step a) and b) in a form of (R)-zopiclone or a mixture of (R)- and (S)- enantiomers of zopiclone enriched in (R)-zopiclone
d) racemisation of the collected (R)-zopiclone or mixture of (R)- and (S)- enantiomers of zopiclone enriched in (R)-zopiclone by treating with an aliphatic amine in an amide solvent or in a mixture of amide solvent and a second solvent,
e) add recovered racemic zopiclone obtained after step d) to the fresh racemic zopiclone in step a).

The term "racemisation" means reducing a relative amount of unwanted enantiomer of zopiclone, preferably (R)-zopiclone, and increasing the relative amount of wanted enantiomer of zopiclone, preferably (S)-zopiclone, to form a racemic zopiclone.

The term "a mixture of (R)- and (S)- enantiomers of zopiclone enriched in (R)-zopiclone" means a mixture of zopiclone enantiomers comprising more than 50% of (R)-zopiclone, preferably more than 70% of (R)-zopiclone, more preferably more than 80% of (R)-zopiclone, even more preferably more than 90% of (R)-zopiclone, most preferably more than 95% of (R)-zopiclone.

The term "racemic zopiclone" means a mixture of (R)- and (S)- enantiomers of zopiclone comprising increased relative amount of (S)-zopiclone in comparison to a starting mixture of (R)- and (S)- enantiomers of zopiclone before racemisation. Preferably the term "racemic zopiclone" means a mixture of (R)- and (S)- enantiomers of zopiclone having an (R)/(S) ratio ranging from about 65/35 to about 35/65, more preferably ranging from about 55/45 to about 45/55, even more preferably ranging from about 52/48 to about 48/52, even more preferably ranging from about 51/49 to about 49/51. Most preferably, the racemic zopiclone has an (R)/(S) ratio of about 1/1.

The amide solvent is selected from *N,N*-dimethylformamide (DMF), N, N-dimethylacetamide (DMA), 1,3-dimethyl-3,4,5,6-tetrahydro-2(1*H*)-pyrimidinone (DMPU), 1,3-dimethyl-2-imidazolidinone, 1-methyl-2-pyrrolidone. The preferred solvent is *N,N*-dimethylformamide.

Amine bases are selected from aliphatic amines such as tri-, di or mono-C₁-C₁₂ alkyl substituted amines in which substituents are the same or different, chained or branched, separated or connected to a cycle as they are but not limited in pyrrolidines piperidines, piperazines, morpholines, unsubstituted or substituted with aryl, or O, N or S containing groups or more cycles as they are but not limited in DABCO (1,4-diazabicyclo[2.2.2]octane). The preferred amines are di- or mono- substituted. The preferred diamines or monoamines are *tert*-butylamine, isobutylamine, piperazine, piperidine, *sec*-butylamine, dipropylamine, diisopropylamine, isopropylamine, the most preferred is isopropylamine. The molar ratio of an amine and zopiclone enantiomer(s) is from 20 mols to 0.2 mol, preferably from 10 mols to 0.5 mol, more preferably from 2 mols to 0.8, most preferably the ratio is 1 mol of amine per 1 mol of zopiclone enantiomer(s).

The optional second solvent is selected from ethanol, isopropanol, ethyl acetate (EtOAc), dichloromethane, diethyl ether, diisopropyl ether, methyl *tert*-butyl ether, water. The most preferable option for the second solvent can be a use of the solvent of the previous process from which the entering material was acquired.

The conversion is carried out above room temperature, preferably in the range from 40 °C to 140 °C, more preferably from 70 °C to 120 °C, most preferably from 85 °C to 95 °C.

Evaporation of the reaction solvent is preferable but it could be limited by technical reasons if the amide solvent has too high boiling point.

If the reaction mixture is diluted with water the solvent for extraction is selected from ethyl acetate, dichloromethane.

If the product of the racemisation according to our invention is used in a continuous industrial process it is preferred to add it to the starting freshly synthesized zopiclone in the ratio between 0 to 100 %. It should be preferably used in the same ratio in each batch, preferably between 5 and 45 %, most preferably between 20 and 30 % of recovered zopiclone in total batch of zopiclone for producing (S)-zopiclone.

The starting unwanted enantiomer can be used as a pure enantiomer or as an enriched mixture in the racemisation process. A pure sample of (R)-zopiclone can be obtained by chromatographic chiral separation of zopiclone from which (S)-zopiclone is finalized but (R)-zopiclone is recovered from (R)-zopiclone fractions, preferably by evaporation of mobile phase under reduced pressure at room temperature.

Preferably, (S)- and (R)-zopiclone are separated on a chiral stationary phase which may comprise amylose tris (3,5-dimethylphenylcarbamate) immobilized on silica gel. Stationary phase comprising silica gel immobilised with functionalized polysaccaride is superior over stationary phase comprising silica gel coated with functionalized polysaccharide due to solvent limitations and general stationary phase stability of the latter. Most preferably, stationary phase comprises Chiralpak^{®} IA.

Although chiral separation of enantiomers of zopiclone had been in our case performed on Chiralpak^{®} AS-V stationary phase we subsequently found out from a comparison of the results on analytical columns (5 µm) that chiral separation on Chiralpak^{®} IA would give better yields due to better capacity and resolution of the column; Rs = 6 (retention time for (R)-zopiclone was 18.5 and for (S)-zopiclone was 24.2) compared to Chiralpak^{®} AS-H; Rs = 3 (retention time for (S)-zopiclone was 4.5 and for (R)-zopiclone was 5.4) when using 96% ethanol as mobile phase. Furthermore, when using Chiralpak^{®} IA peaks of both enantiomers were well separated from the peak of the solvent (retention times for amide solvents were around 4.0) used in the racemisation process which enabled monitoring of the racemisation in the reaction mixture. This was impossible in the case of old HPLC method on Chiralpak^{®} AS-H since the peak of the solvent overlapped with the peak of (S)-zopiclone.

Preferably, the mobile phase used in the separation of (S)- and (R)-zopiclone comprises an C₁-C₆ alcohol, tetrahydrofuran, methyl *tert*-butyl ether, dichloromethane or a mixture thereof. The C₁-C₆ alcohol may comprise methanol, ethanol, 2-propanol or a mixture thereof. When using alcohol as the mobile phase, optionally water may be used as a co-solvent. Most preferably, 96 % ethanol is used as a mobile phase in the chiral chromatographic separation.

Another preferred embodiment of the present invention is a process for racemisation of (R)-zopiclone comprising the following steps:
a) providing a material comprising (R)-zopiclone by recovering (R)-zopiclone from (R)-zopiclone fractions obtained after a separation of racemic zopiclone by means of chromatographic chiral separation, wherein a chiral stationary phase is silica gel immobilised with functionalized polysaccharide, preferably amylose tris (3,5-dimethylphenylcarbamate),
b) treating the material with an aliphatic amine in an amide solvent or in a mixture of amide solvent and a second solvent,
c) isolating a racemic zopiclone.
   Another more preferred embodiment of the present invention is a process for racemisation of (R)-zopiclone comprising the following steps:
   a) providing a material comprising (R)-zopiclone by recovering (R)-zopiclone from (R)-zopiclone fractions obtained after a separation of racemic zopiclone by means of chromatographic chiral separation, wherein a chiral stationary phase is silica gel immobilised with functionalized polysaccharide, preferably amylose tris (3,5-dimethylphenylcarbamate), and mobile phase is selected from C₁-C₆ alcohol, tetrahydrofuran, methyl *tert*-butyl ether, dichloromethane or a mixture thereof or a mixture of a C₁-C₆ alcohol with water, preferably a mobile phase is 96% ethanol,
   b) treating the material with an aliphatic amine in an amide solvent or in a mixture of amide solvent and a second solvent,
   c) isolating a racemic zopiclone.
      Another preferred embodiment of the present invention is a continuous process for preparing (S)-zopiclone comprising the following steps:
      a) separation of racemic zopiclone by means of chromatographic chiral separation, wherein a chiral stationary phase is silica gel immobilised with functionalized polysaccharide, preferably amylose tris (3,5-dimethylphenylcarbamate),
      b) isolation of (S)-zopiclone,
      c) racemisation of (R)-zopiclone recovered from (R)-zopiclone fractions obtained in step a) by treating with an aliphatic amine in an amide solvent or in a mixture of amide solvent and a second solvent,
      d) optionally repeating step c),
      e) add recovered racemic zopiclone obtained after step c) or step d) to the fresh racemic zopiclone in step a).

Another preferred embodiment of the present invention is a continuous process for preparing (S)-zopiclone comprising the following steps:
a) separation of racemic zopiclone by means of chromatographic chiral separation, wherein a chiral stationary phase is silica gel immobilised with functionalized polysaccharide, preferably amylose tris (3,5-dimethylphenylcarbamate),
b) isolation of (S)-zopiclone,
c) racemisation of (R)-zopiclone recovered from (R)-zopiclone fractions obtained in step a) by treating with an aliphatic amine in an amide solvent or in a mixture of amide solvent and a second solvent,
d) add recovered racemic zopiclone obtained after step c) to the fresh racemic zopiclone in step a).

Another more preferred embodiment of the present invention is a continuous process for preparing (S)-zopiclone comprising the following steps:
a) separation of racemic zopiclone by means of chromatographic chiral separation, wherein a chiral stationary phase is silica gel immobilised with functionalized polysaccharide, preferably amylose tris (3,5-dimethylphenylcarbamate), and mobile phase is selected from C₁-C₆ alcohol, tetrahydrofuran, methyl *tert*-butyl ether, dichloromethane or a mixture thereof or a mixture of a C₁-C₆ alcohol with water, preferably a mobile phase is 96% ethanol,
b) isolation of (S)-zopiclone,
c) racemisation of (R)-zopiclone recovered from (R)-zopiclone fractions obtained in step a) by treating with an aliphatic amine in an amide solvent or in a mixture of amide solvent and a second solvent,
d) optionally repeating step c),
e) add recovered racemic zopiclone obtained after step c) or step d) to the fresh racemic zopiclone in step a).

Another more preferred embodiment of the present invention is a continuous process for preparing (S)-zopiclone comprising the following steps:
a) separation of racemic zopiclone by means of chromatographic chiral separation, wherein a chiral stationary phase is silica gel immobilised with functionalized polysaccharide, preferably amylose tris (3,5-dimethylphenylcarbamate), and mobile phase is selected from C₁-C₆ alcohol, tetrahydrofuran, methyl *tert*-butyl ether, dichloromethane or a mixture thereof or a mixture of a C₁-C₆ alcohol with water, preferably a mobile phase is 96% ethanol,
b) isolation of (S)-zopiclone,
c) racemisation of (R)-zopiclone recovered from (R)-zopiclone fractions obtained in step a) by treating with an aliphatic amine in an amide solvent or in a mixture of amide solvent and a second solvent,
d) add recovered racemic zopiclone obtained after step c) to the fresh racemic zopiclone in step a).

In one scope the invention provides a process for the separation of (S)- and (R)-zopiclone by means of chiral chromatography using silica gel immobilized with a functionalized polysaccharide (Chiralpak^{®} IA) as stationary phase. The use of Chiralpak^{®} IA as such enables separation of enantiomers of zopiclone in samples contaminated with residual solvents arising from racemisation or isolation processes.

Another possibility for acquiring of pure (R)-zopiclone or (R)-zopiclone with slight amount of (S)-zopiclone, is a separation of the unwanted isomer by crystallisation of diasteroisomeric salts like L-malate. In such a process (S)-zopiclone is obtained by purification of crystallisation liquor by several recrystallisations or by any other process. This option is much less convenient for providing the wanted (S)-isomer.

The most often application of zopiclone racemisation is recovering of mother liquors after separation of (S)-zopiclone by crystallisation of diasteroisomeric salts. In such case mother liquors contains mixtures of both enantiomers in the form of a salt which are usually but not necessary further converted into the mixture of enantiomers in the form of bases. Such mixtures are enriched in (R)-zopiclone and consist of 51-100 % of (R)-zopiclone, preferably 70-90 % of (R)-zopiclone, most preferably 77-87 % of (R)-zopiclone.

Thus, racemic zopiclone is converted to a salt with a chiral acid which favours such crystal packaging of diastereoisomeric salts that one enantiomer of zopiclone is far preferable over the mixtures of enantiomers or racemates. Such acids are preferably selected from organic acids, such as malic acid, or substituted organic acids, preferably O,O-substituted tartaric acid, most preferably O,O-dibenzoyltartaric acid or N-substituted amino acids such as Z-phenylalanine. In a preferred option zopiclone is treated with D-malic acid in a mixture of methanol and acetone. After heating, preferably to 55 °C the reaction mixture is gradually cooled bellow room temperature, preferably to 10-15 °C under moderate stirring (max. 150 rpm) and upon seeding preferably at 45-50 °C. Resulting slurry is held at 10-15 °C for additional 30 minutes, then the product is isolated by filtration and washed with cold methanol to yield wet (S)-zopiclone D-malate (40-45 %, 95-97 % ee) and mother liquors with in (R)-enriched zopiclone D-malate (50-80 % ee).

Wet (S)-zopiclone D-malate is suspended in a water non miscible solvent, preferably ethyl acetate and water. Reaction mixture is heated to 30-40 °C and aqueous K₂CO₃ solution is added slowly and mixture heated to 60-65 °C in order to get clear solution. The organic phase is isolated and washed with warm water (50-60 °C). The mixture is polish filtered, rinsed with ethyl acetate, concentrated under reduced pressure and heated to reflux to get clear solution, filtered and cooled to 0 °C in 2 h under stirring to precipitate crystals which are isolated by filtration. The crystalline product is dried at 40 °C over 8-20 h to yield (S)-zopiclone (72-78 %, 99.8% ee). Total yield from both steps is 31-37 %.

Mother liquors with in (R)-enriched zopiclone D-malate (50-80 % ee) is concentrated under reduced pressure and afterwards antisolvent, preferably diethyl ether is added. The resulting slurry is cooled to 0 °C and isolated by filtration to get wet product (50-60 %, 50-80 % ee (R)-zopiclone as D-malate), which is charged into reactor. A water immiscible solvent, preferably ethyl acetate and water are added and the reaction mixture is heated to 30-40 °C. An aqueous K₂CO₃ solution is added slowly over 5 minutes. The mixture is heated to 60-65°C to get clear solution and the organic phase is isolated and washed with warm water (50-60 °C). The mixture is polish filtered, rinsed with ethyl acetate and concentrated. Into the residue antisolvent, preferably diethyl ether is added. The resulting slurry is cooled to 0 °C, isolated by filtration and dried at 40 °C to get with (R)-enantiomer enriched zopiclone (95 %, 50-80 % ee).

A mixture of zopiclone enriched with (R)-enantiomer (50-80 % ee), amide solvent, preferably DMF and primary or secondary amine, preferably isopropylamine (equimolar quantity regarding zopiclone) are added. Reaction mixture is heated at 85 °C. Optionally, if a volatile such as isopropylamine is used every hour an additional amount of amine is added. After 3-8 hours reaction mixture is concentrated and poured into water at room temperature under vigorous stirring. Resulting slurry is stirred for additional hour, filtered, washed with water and dried to get racemic recovered zopiclone (70-80 %, 97-98 % of chromatographic purity).

Recovered zopiclone (97-98 % of chromatographic purity) prepared by racemisation of (R)-enantiomer can be purified by crystallisation from methanol at 0 °C to get purified racemic zopiclone (60-70 %) with 99 % chromatographic purity. Total yield of recovered purified zopiclone is 22-28 %.

The procedure of the invention recovers 22-28 % of racemic zopiclone which enters again in the separation process. The yield of (S)-zopiclone is therefore enhanced from 31-37 % to 38-44 % in one cycle. The obtained recovered zopiclone is further submitted to repeating separation of enantiomers what after mathematical approximation upon several cycles enhances the yield of (S)-zopiclone to 44-50 %.

Another preferred embodiment of the present invention is a process for racemisation of (R)-zopiclone comprising the following steps:
a) providing a material comprising (R)-zopiclone by recovering (R)-zopiclone from a mother liquor obtained after separation of (S)-zopiclone diasteroisomeric salt,
b) treating the material with an aliphatic amine in an amide solvent or in a mixture of amide solvent and a second solvent,
c) isolating a racemic zopiclone.

Another more preferred embodiment of the present invention is a process for racemisation of (R)-zopiclone comprising the following steps:
a) providing a material comprising (R)-zopiclone by recovering (R)-zopiclone diasteroisomeric salt from a mother liquor obtained after separation of (S)-zopiclone diasteroisomeric salt and converting (R)-zopiclone diasteroisomeric salt to (R)-zopiclone free base,
b) treating the material with an aliphatic amine in an amide solvent or in a mixture of amide solvent and a second solvent,
c) isolating a racemic zopiclone.

Another preferred embodiment of the present invention is a continuous process for preparing (S)-zopiclone comprising the following steps :
a1) reacting racemic zopiclone with a chiral acid, preferably selected from D-malic acid, O,O-substituted tartaric acid, N-substituted amino acids, more preferably selected from D-malic acid, O,O-dibenzoyltartaric acid, Z-phenylylanine, most preferably D-malic acid,
a2) isolation of (S)-zopiclone diasteroisomeric salt obtained in sub-step a1) by crystallisation,
a3) conversion of (S)-zopiclone diasteroisomeric salt to (S)-zopiclone free base.
b) isolation of (S)-zopiclone,
c) racemisation of a mixture of (R)- and (S)- enantiomers of zopiclone enriched in (R)-zopiclone obtained from a mother liquor obtained after separation of (S)-zopiclone diasteroisomeric salt in step a2) and conversion (R)- and (S)- zopiclone diasteroisomeric salts to (R)- and (S)- zopiclone free base, by treating the mixture of (R)- and (S)- enantiomers of zopiclone enriched in (R)-zopiclone with an aliphatic amine in an amide solvent or in a mixture of amide solvent and a second solvent,
d) optionally repeating step c),
e) add recovered racemic zopiclone obtained after step c) or step d) to the fresh racemic zopiclone in step a).

Another preferred embodiment of the present invention is a continuous process for preparing (S)-zopiclone comprising the following steps :
a1) reacting racemic zopiclone with a chiral acid, preferably selected from D-malic acid, O,O-substituted tartaric acid, N-substituted amino acids, more preferably selected from D-malic acid, O,O-dibenzoyltartaric acid, Z-phenylylanine, most preferably D-malic acid,
a2) isolation of (S)-zopiclone diasteroisomeric salt obtained in sub-step a1) by crystallisation,
a3) conversion of (S)-zopiclone diasteroisomeric salt to (S)-zopiclone free base.
b) isolation of (S)-zopiclone,
c) racemisation of a mixture of (R)- and (S)- enantiomers of zopiclone enriched in (R)-zopiclone obtained from a mother liquor obtained after separation of (S)-zopiclone diasteroisomeric salt in step a2) and conversion (R)- and (S)- zopiclone diasteroisomeric salts to (R)- and (S)- zopiclone free base, by treating the mixture of (R)- and (S)- enantiomers of zopiclone enriched in (R)-zopiclone with an aliphatic amine in an amide solvent or in a mixture of amide solvent and a second solvent,
d) add recovered racemic zopiclone obtained after step c) to the fresh racemic zopiclone in step a).

The following examples are offered to illustrate aspects of the present invention, and are not intended to limit or define the present invention in any manner.

Example 1: Preparation of (S)-zopiclone:
A three-neck 3.0 L flask was charged with zopiclone (50 g, 0.126 mol), D-malic acid (16.7 g, 0.125 mol), 508 mL MeOH and 945 mL acetone. The reaction mixture was heated to 55-56 °C for about 30 min and gradually cooled to 45-47 °C over approximately 30 minutes. (S)-zopiclone D-malate seeds (0.18 g, 0.3 %) were added at 45-47 °C. The reaction mixture was cooled to 40 °C over 1 h and then cooled to 10-15 °C over 3 h. The slurry was held at 10-15 °C for 30 minutes. The product was isolated by filtration, washed with 150 mL of cold methanol and dried at 40 °C over 6-16 h to give (S)-zopiclone D-malate (27.0 g, 41 %, 98.5 % ee)

A three-neck 1 L flask was charged with (S)-zopiclone D-malate (26.0 g, 98.5 % ee), 52 mL of water and 390 mL of EtOAc. The reaction mixture was heated in an oil bath to 30-40 °C. An aqueous K₂CO₃ solution (40 %, 20.8 g) was added slowly over 5 min. The mixture was then heated to 60-65 °C and the organic phase was isolated and washed with 260 mL water. The mixture was polish filtered, rinsed with EtOAc (20 mL) and concentrated to 95-105 mL. The resulting slurry was cooled and held for 2 h at 0-5 °C. The crystal product was isolated by filtration and washed with cold EtOAc (50 mL). The white product was dried at 40 °C over 8-20 h to give (S)-zopiclone (16.0 g, 83 %, 100 % ee).
Total yield = 34 %

### Example 2: Preparation of (R)-zopiclone

A three-neck 500 mL flask was charged with zopiclone (10 g, 26 mmol), L-malic acid (3.3 g, 25 mmol), 102 mL MeOH and 190 mL acetone. The reaction mixture was heated to 55-56 °C for about 30 min and gradually cooled to 45-47 °C over approximately 30 minutes. (R)-zopiclone L-malate seeds were added at 45-47 °C. The reaction mixture was cooled to 40 °C over 40 minutes and then cooled to 10 °C over 1 hour and 30 minutes. The slurry was held at 10 °C for 1 hour. The product was isolated by filtration, washed with 40 mL of cold methanol and dried at 40 °C over 10-20 h to give (R)-zopiclone L-malate (5.7 g, 43 %, 95.9 % ee).

A three-neck 250 mL flask was charged with (R)-zopiclone L-malate (5.6 g, 95.9% ee), 11 mL of water, 42 mL of EtOAc and 42 mL of dichloromethane. The reaction mixture was heated in an oil bath to 30-40 °C. An aqueous K₂CO₃ solution (40 %, 4.5 g) was added slowly over 5 minutes. The mixture was then heated to 60-65 °C and the organic phase was isolated and washed with 56 mL water. The mixture was polish filtered, rinsed with EtOAc (10 mL) and concentrated to 40-45 mL. The resulting slurry was cooled and held for 2 h at 0-5 °C. The crystal product was isolated by filtration and washed with cold EtOAc (20 mL). The white product was dried at 40 °C over 8-20 h to give (R)-zopiclone (3.4 g, 82 %, 99.8 % ee).
Total yield = 35 %.

### Example 3: Separation of (S)- and (R)-zopiclone by HPLC preparative chromatography

(S)- and (R)-zopiclone were separated by means of preparative chromatography using Chiralpak^{®} AS-V (20 µm) as a stationary phase and 96% ethanol as a mobile phase (volume of injection: 40 ml; 6 g of zopiclone per liter of mobile phase; flow rate: 100 mL/min). Retention time for (S)-zopiclone was 14 minutes and for (R)-zopiclone was 17 minutes.

Collected fractions of (S)-zopiclone were evaporated under reduced vacuum at room temperature and dried at 50 °C over 17 hours in order to get 20 g of (S)-zopiclone (yield = 36 %, 99.36 % ee).

Collected fractions of (R)-zopiclone were concentrated under reduced vacuum at room temperature, crystallized from i-PrOH at -20 °C. Precipitated crystals were isolated and dried at 40 °C over 4 hours in order to get 13 g of (R)-zopiclone (yield = 24 %, 88.00 % ee).

### Example 4: Racemisation of (R)-zopiclone in DMF/Et₃N

(R)-zopiclone (200 mg, 0.5 mmol) from Example 3 and Et₃N (1 mL, 7.2 mmol) were heated at 120 °C under stirring in 20 mL of DMF. After 2 hours of heating dark brown solution was obtained with poor conversion of (R)-zopiclone into (S)-zopiclone (17 %) and increase of impurities was observed by HPLC analysis.

### Example 5: Racemisation of (R)-zopiclone in DMF/Et₃N/H₂O

A mixture (200 mg, 0.5 mmol) of both enantiomers of zopiclone (20 % of (S)-zopiclone and 80 % of (R)-zopiclone), Et₃N (1 mL, 7.2 mmol) was heated at 120 °C under stirring in a mixture of 5 mL of DMF and 1 mL of H₂O. After 4 hours and 30 minutes of heating orange coloured solution was obtained with ratio of 46 % of (S)-zopiclone and 54 % of (R)-zopiclone, unfortunately increase of impurities was observed by HPLC analysis.

If the procedure is repeated with 1 equivalent of triethylamine no racemisation takes place.

### Example 6: Racemisation of (R)-zopiclone in DMF/DBU (1,8-diazabicyclo[5.4.0]undec-7-ene)

(R)-zopiclone (200 mg, 0.5 mmol) from Example 3 and DBU (0.3 mL, 2 mmol) were heated at 120 °C under stirring in 20 mL of DMF. After 30 minutes of heating dark brown solution with complete degradation of the product was observed and confirmed by HPLC analysis.

### Example 7: Racemisation of (R)-zopiclone in DMF/N-ethyldiisopropylamine

(R)-zopiclone (200 mg, 0.5 mmol) from Example 3 and N-etildiizopropilamine (0.3 mL, 1.7 mmol) were heated at 120 °C under stirring in 20 mL of DMF. After 3 hours of heating orange coloured solution was obtained with no conversion of (R)-zopiclone into (S)-zopiclone was observed by chiral HPLC analysis.

### Example 8: Racemisation of (R)-zopiclone in DMF/dicyclohexylamine

(R)-zopiclone (200 mg, 0.5 mmol) from Example 3 and dicyclohexylamine (0.3 mL, 1.5 mmol) were heated at 120 °C under stirring in 20 mL of DMF. After 3 hours of heating orange coloured solution was obtained with poor conversion of (R)-zopiclone into (S)-zopiclone (16 %).

### Example 9: Racemisation of (R)-zopiclone in DMF/4-dimethylaminopyridine

(R)-zopiclone (200 mg, 0.5 mmol) from Example 3 and 4-dimethylaminopyridine (50 mg, 0.4 mmol) were heated at 135 °C under stirring in 20 mL of DMF. After 6 hours of heating yellow coloured solution was obtained with poor conversion of (R)-zopiclone into (S)-zopiclone (12 %).

### Example 10: Racemisation of (R)-zopiclone in DMF/DABCO

(R)-zopiclone (200 mg, 0.5 mmol) from Example 3 and DABCO (100 mg, 0.9 mmol) were heated at 135 °C under stirring in 10 mL of DMF. After 6 hours of heating yellow solution was obtained containing 44.25 % of (S)-zopiclone and 55.75 % of (R)-zopiclone. Reaction mixture was concentrated under vacuum at 80 °C. Into the residue 50 mL of water was added and pH adjusted to 7 with 0.05 M HCl. Racemized product was extracted with EtOAc (2 x 30 mL). Combined organic phases were concentrated to 5-10 mL and left in the refrigerator overnight. Precipitated crystals were isolated by filtration and dried at 40 °C under vacuum to yield 110 mg of product (55 %, 0.36 % ee (R)-zopiclone).

### Example 11: Racemisation of (R)-zopiclone in DMF/DABCO

A mixture (8.1 g, 20.8 mmol) of both enantiomers of zopiclone (12.5 % of (S)-zopiclone and 87.5 % of (R)-zopiclone) and DABCO (0.8 g, 6.6 mmol) were heated at 125 °C under stirring in 120 mL of DMF. After 4 hours of heating yellow solution was obtained containing 43.29 % of (S)-zopiclone and 56.71 % of (R)-zopiclone. Reaction mixture was concentrated to app. 10 mL under vacuum at 80 °C. Into the residue 100 mL of water was added under stirring and pH adjusted to 7 with 0.05 M HCl. After stirring 10 minutes precipitated product isolated by filtration and dried at 40 °C under vacuum to yield 6.9 g of product (85 %, 7.2 % ee (R)-zopiclone).

### Example 12: Racemisation of (R)-zopiclone in DMF/tert-butylamine

A mixture (200 mg, 0.5 mmol) of both enantiomers of zopiclone (20 % of (S)-zopiclone and 80 % of (R)-zopiclone) and *tert*-butylamine (1 mL, 9.5 mmol) were heated at 125 °C under stirring in 10 mL of DMF. After 2 hours of heating yellow solution was obtained containing 48.42 % of (S)-zopiclone and 51.58 % of (R)-zopiclone as determined by chiral HPLC. The product was not isolated.

### Example 13: Racemisation of (R)-zopiclone in DMF/isobutylamine

A mixture (200 mg, 0.5 mmol) of both enantiomers of zopiclone (20 % of (S)-zopiclone and 80 % of (R)-zopiclone) and isobutylamine (1 mL, 10 mmol) were heated at 125 °C under stirring in 10 mL of DMF. After 30 minutes of heating yellow solution was obtained containing 49.45 % of (S)-zopiclone and 50.55 % of (R)-zopiclone as determined by chiral HPLC. The product was not isolated.

### Example 14: Racemisation of (R)-zopiclone in DMF/piperazine

A mixture (200 mg, 0.5 mmol) of both enantiomers of zopiclone (20 % of (S)-zopiclone and 80 % of (R)-zopiclone) and piperazine (0.8 g, 9.3 mmol) were heated at 125 °C under stirring in 10 mL of DMF. After 30 minutes of heating yellow solution was obtained containing 47.96 % of (S)-zopiclone and 52.04 % of (R)-zopiclone as determined by chiral HPLC. The product was not isolated.

### Example 15: Racemisation of (R)-zopiclone in DMF/piperidine

A mixture (200 mg, 0.5 mmol) of both enantiomers of zopiclone (20 % of (S)-zopiclone and 80 % of (R)-zopiclone) and piperidine (1 mL, 10 mmol) were heated at 125 °C under stirring in 10 mL of DMF. After 30 minutes of heating yellow solution was obtained containing 50.03 % of (S)-zopiclone and 49.97 % of (R)-zopiclone as determined by chiral HPLC. The product was not isolated.

### Example 16: Racemisation of (R)-zopiclone in DMF/sec-butylamine

A mixture (200 mg, 0.5 mmol) of both enantiomers of zopiclone (20 % of (S)-zopiclone and 80 % of (R)-zopiclone) and sec-butylamine (1 mL g, 9.9 mmol) were heated at 125 °C under stirring in 10 mL of DMF. After 30 minutes of heating yellow solution was obtained containing 44.85 % of (S)-zopiclone and 55.15 % of (R)-zopiclone as determined by chiral HPLC. The product was not isolated.

### Example 17: Racemisation of (R)-zopiclone in DMF/dipropylamine

A mixture (200 mg, 0.5 mmol) of both enantiomers of zopiclone (20 % of (S)-zopiclone and 80 % of (R)-zopiclone) and dipropylamine (1.2 mL g, 7.3 mmol) were heated at 125 °C under stirring in 10 mL of DMF. After 1 hour of heating yellow solution was obtained containing 48.88 % of (S)-zopiclone and 51.12% of (R)-zopiclone as determined by chiral HPLC. The product was not isolated.

### Example 18: Racemisation of (R)-zopiclone in DMF/dipropylamine

A mixture (10 g, 26 mmol) of both enantiomers of zopiclone (20 % of (S)-zopiclone and 80 % of (R)-zopiclone) and dipropylamine (3.5 mL g, 26 mmol) were heated at 120 °C under stirring in 150 mL of DMF. After 3 hours and 30 minutes of heating yellow solution was obtained containing 45.00 % of (S)-zopiclone and 55.00 % of (R)-zopiclone as determined by chiral HPLC. The reaction mixture was concentrated under reduced vacuum to approximately 15 mL, cooled to 0 °C and 20 mL of diethyl ether was added under starring. Precipitated product was isolated by filtration and dried to yield 4.9 g of zopiclone (49 %) with slight excess of (R)-zopiclone (9 % ee).

### Example 19: Racemisation of (R)-zopiclone in DMF/diisopropylamine

A mixture (5 g, 13 mmol) of both enantiomers of zopiclone (20 % of (S)-zopiclone and 80 % of (R)-zopiclone) and diisopropylamine (1.8 mL g, 13 mmol) were heated at 110 °C under stirring in 70 mL of DMF. After 6 hours and 30 minutes of heating yellow solution was obtained containing 40.78 % of (S)-zopiclone and 59.22 % of (R)-zopiclone as determined by chiral HPLC. The product was not isolated.

### Example 19: Racemisation of (R)-zopiclone in DMF/isopropylamine

A mixture (5 g, 13 mmol) of both enantiomers of zopiclone (20 % of (S)-zopiclone and 80 % of (R)-zopiclone) and isopropylamine (1.1 mL g, 13 mmol) were heated at 110 °C under stirring in 70 mL of DMF. After 2 hours and 30 minutes of heating yellow solution was obtained containing 48.49 % of (S)-zopiclone and 51.51 % of (R)-zopiclone as determined by chiral HPLC. The reaction mixture was concentrated under reduced vacuum to approximately 20 mL, cooled to 0 °C and 20 mL of diethyl ether was added under starring. Precipitated product was isolated by filtration and dried to yield 3.6 g of zopiclone (49 %) with slight excess of (R)-zopiclone (2 % ee).

### Example 20: Racemisation of (S)-zopiclone in DMF/isopropylamine

(S)-zopiclone (1.0 g, 2.6 mmol) from Example 1 and isopropylamine (0.22 mL g, 2.6 mmol) were heated at 85 °C under stirring in 12 mL of DMF. After 6 hours of heating yellow solution was obtained containing 57.37 % of (S)-zopiclone and 42.63 % of (R)-zopiclone as determined by chiral HPLC. The product was not isolated.

### Example 21: Racemisation of (R)-zopiclone in DMA/isopropylamine

(R)-zopiclone (830 mg, 2.2 mmol) from Example 3 and isopropylamine (0.18 mL g, 2.2 mmol) were heated at 85 °C under stirring in 10 mL of DMA. After 6 hours of heating yellow solution was obtained containing 44.60 % of (S)-zopiclone and 55.40 % of (R)-zopiclone as determined by chiral HPLC. The product was not isolated.

### Example 22: Racemisation of (R)-zopiclone in DMPU/isopropylamine

A mixture (830 mg, 2.2 mmol) of both enantiomers of zopiclone (20 % of (S)-zopiclone and (80 % of (R)-zopiclone) and isopropylamine (0.18 mL g, 2.2 mmol) were heated at 85 °C under stirring in 10 mL of DMPU. After 6 hours of heating yellow solution was obtained containing 46.22 % of (S)-zopiclone and 53.78 % of (R)-zopiclone as determined by chiral HPLC. The product was not isolated.

Example 23: Racemisation of (R)-zopiclone in 1-methyl-2-pyrrolidone/isopropylamine (R)-zopiclone (830 mg, 2.2 mmol) from Example 3 and isopropylamine (0.18 mL g, 2.2 mmol) were heated at 85 °C under stirring in 10 mL of 1-methyl-2-pyrrolidone. After 6 hours of heating yellow solution was obtained containing 44.83 % of (S)-zopiclone and 55.17 % of (R)-zopiclone as determined by chiral HPLC. The product was not isolated.

### Example 24: Racemisation of (R)-zopiclone in 1,3-dimethyl-2-imidazolidinone/isopropylamine

(R)-zopiclone (830 mg, 2.2 mmol) from Example 3 and isopropylamine (0.18 mL g, 2.2 mmol) were heated at 85 °C under stirring in 10 mL of 1 ,3-dimethyl-2-imidazolidinone. After 6 hours of heating yellow solution was obtained containing 39.66 % of (S)-zopiclone and 60.34 % of (R)-zopiclone as determined by chiral HPLC. The product was not isolated.

### Example 25: Preparation of (S)-zopiclone and conversion of (R)-zopiclone into racemic zopiclone

A 5 L reactor was charged with 100 g (0.25 mol) of zopiclone (90 g of zopiclone according to Example 1 with 10 g of recovered zopiclone, prepared according to Example 19), D-malic acid (33.5 g, 0.25 mol), 1015 mL MeOH and 1885 mL acetone. The reaction mixture was heated to 55-56 °C for about 30 minutes and gradually cooled to 45-47 °C over approximately 30 min. (S)-zopiclone D-malate seeds (0.25 g, 0.2%) were added at 45-47 °C under stirring at max. 150 rpm. The reaction mixture was cooled to 40 °C over 1 h and then cooled to 10-15 °C over 3 h. Then the slurry was held at 10-15 °C for 30 minutes. The product was isolated by filtration, the cake washed with cold methanol (2 x 150 mL) to give approximately 104 g of wet (S)-zopiclone D-malate (56 g of dry product, 43 %, 95.8 % ee) and approximately 2.6 L of filtrate with (R)-enriched zopiclone D-malate.

A 5 L reactor was charged with 101 g of wet (S)-zopiclone D-malate (54 g calculated on dry product), 120 mL of water and 1200 mL of EtOAc. The reaction mixture was heated to 30-40 °C. An aqueous K₂CO₃ solution (40 %, 43 g) was added slowly over 5 minutes. The mixture was then heated to 60-65 °C and the organic phase was isolated and washed with 560 mL of water heated to 55 °C. The mixture was polish filtered, rinsed with EtOAc (120 mL) and concentrated to 750 mL, heated to reflux to get clear solution, filtered and cooled to 0 °C in 2 h under stirring to precipitate product which was isolated by filtration and washed with cold EtOAc (120 mL). The crystalline product was dried at 40 °C over 8-20 h to yield (S)-zopiclone (30 g, 75 %, 99.80 %ee).

2.6 L of filtrate with (R)-enriched zopiclone D-malate was concentrated to 150 mL, 400 mL of diethyl ether was added. The resulting slurry was chilled to 0 °C and isolated by filtration to get 125 g of wet product (74 g of dry product, 55%, 67.44 % ee (R)-zopiclone), which was charged into 5 L reactor. EtOAC (1400 mL) and water (160 mL) were added and the reaction mixture was heated to 30-40 °C. An aqueous K₂CO₃ solution (40 %, 58 g) was added slowly over 5 minutes. The mixture was then heated to 60-65°C and the organic phase was isolated and washed with 630 mL of water heated to 55 °C. The mixture was polish filtered, rinsed with EtOAc (170 mL), concentrated to 250 mL. Into the residue 400 mL of diethyl ether was added. The resulting slurry was chilled to 0 °C, isolated by filtration and dried at 40 °C to get 52 g of with (R)-enantiomer enriched zopiclone (95 %, 67.44 % ee (R)-zopiclone).

A 2L flask was charged with 50 g (0.13 mol) of with (R)-enantiomer enriched zopiclone, 630 mL of DMF and 11.2 mL of isopropylamine. Reaction mixture was heated at 85 °C. Every hour and a half additional 2.5 mL (0.03 mol) of isopropylamine were added. After 5 hours and a half reaction mixture was concentrated to 30-40 mL and poured into 750 mL of water at room temperature under vigorous stirring. Resulting slurry was stirred for additional hour, filtered, washed with water (2 x 150 mL) and dried to get 38.7 g of racemic zopiclone (77 %, 97.6 % chromatographic purity).

Zopiclone (36 g) prepared by racemisation of (R)-enantiomer can be purified by crystallisation from 500 mL of MeOH at 0°C to get 22 g of racemic zopiclone (60%) with 99% chromatographic purity.

## Claims

1. A process for racemisation of an enantiomer of zopiclone or enantiomerically enriched mixture of (R)- and (S)- enantiomers of zopiclone by treating with an aliphatic amine in an amide solvent or in a mixture of amide solvent and a second solvent.

2. A process for racemisation of (R)-zopiclone comprising the following steps:
a) providing a material comprising (R)-zopiclone,
b) treating the material with an aliphatic amine in an amide solvent or in a mixture of amide solvent and a second solvent,
c) isolating a racemic zopiclone.

3. A process according to any of the preceding claims, wherein the aliphatic amine is selected from the group consisting of mono-, di- or tri-C₁-C₁₂ alkyl substituted amines in which substituents are the same or different, chained or branched, separated or connected to a cycle as they are but not limited in pyrrolidines, piperidines, piperazines, morpholines, unsubstituted or substituted with aryl, or O, N or S containing groups or more cycles, preferably the aliphatic amine is mono- or di-C₁-C₁₂ alkyl substituted amine.

4. A process according to any of the preceding claims, wherein the molar ratio of the aliphatic amine and zopiclone enantiomer(s) is 20:0.2, preferably 10:0.5 mol, more preferably 2:0.8, most preferably 1:1.

5. A process according to any of the preceding claims, wherein the amide solvent is selected from N,N-dimethylformamide, N, N-dimethylacetamide, 1,3-dimethyl-3,4,5,6-tetrahydro-2(1 H)-pyrimidinone, 1,3-dimethyl-2-imidazolidinone, 1-methyl-2-pyrrolidone, preferably the amide solvent is N,N-dimethylformamide.

6. A process for preparing (S)-zopiclone comprising a process for racemisation of (R)-zopiclone or a mixture of (R)- and (S)- enantiomers of zopiclone enriched in (R)-zopiclone according to any of the preceding claims.

7. A continuous process for preparing (S)-zopiclone comprising the following steps:
a) separation of racemic zopiclone,
b) isolation of (S)-zopiclone,
c) collecting the remaining zopiclone from step a) and b) in a form of (R)-zopiclone or a mixture of (R)- and (S)- enantiomers of zopiclone enriched in (R)-zopiclone
d) racemisation of the collected (R)-zopiclone or mixture of (R)- and (S)- enantiomers of zopiclone enriched in (R)-zopiclone by treating with an aliphatic amine in an amide solvent or in a mixture of amide solvent and a second solvent according to any of claims 1 to 5,
e) optionally repeating step d),
f) add recovered racemic zopiclone obtained after step d) or step e) to the fresh racemic zopiclone in step a).

8. A process according to claim 7, wherein the separation of racemic zopiclone is carried out by chromatographic chiral separation.

9. A process according to claim 7, wherein the step a) comprises the following sub-steps:
a1) reacting racemic zopiclone with a chiral acid, preferably selected from D-malic acid, O,O-substituted tartaric acid, N-substituted amino acids, more preferably selected from D-malic acid, O,O-dibenzoyltartaric acid, Z-phenylylanine, most preferably D-malic acid,
a2) isolation of (S)-zopiclone diasteroisomeric salt obtained in sub-step a1) by crystallisation,
a3) conversion of (S)-zopiclone diasteroisomeric salt to (S)-zopiclone free base

10. A process for the separation of racemic zopiclone by means of chromatographic chiral separation, wherein a chiral stationary phase is silica gel immobilised with functionalized polysaccharide, preferably amylose tris (3,5-dimethylphenylcarbamate).
